# EUROPEAN PATENT SPECIFICATION

(11) **EP 1 639 946 B1**
(45) Date of publication and mention of the grant of the patent: **14.11.2007**
(21) Application number: 04022856.1
(22) Date of filing: 24.09.2004
(51) Int. Cl.: A61B 8/08

(54) **Ultrasonic diagnostic apparatus**
Ultraschall-Diagnosegerät
Dispositif de diagnostic ultrasonore

(43) Date of publication of application: 29.03.2006
(73) Proprietor: Aloka Co. Ltd., Mitaka-shi Tokyo, 181-8622 (JP); Nakamura, Kozo, Tokyo, 179-0081 (JP); Ohnishi, Isao, Tokyo 166-0001 (JP)
(72) Inventor: Sakai, Ryoichi, Mitaka-shi Tokyo 181-8622 (JP); Harada, Akimitsu, Mitaka-shi Tokyo 181-8622 (JP); Nakamura, Kozo, Tokyo, 179-0081 (JP); Ohnishi, Isao, Tokyo, 166-0001 (JP); Matsuyama, Jyuntaro 202 Ichigaya Ousaka House, Shinjuku-ku, Tokyo 162-0842 (JP); Uehara, Toshiro, Tokyo, 113-0033 (JP)
(74) Representative: Heim, Hans-Karl

(56) References cited:
- EP-A- 0 737 441
- US-A- 5 368 044
- US-A- 5 474 070
- US-A- 5 678 565
- US-A1- 2002 103 432

## Description

### BACKGROUND OF THE INVENTION

### 1. Field of the Invention

The present invention relates to an ultrasonic diagnostic apparatus and in particular to an ultrasonic diagnostic apparatus for evaluating mechanical characteristics of bone.

### 2. Description of the Related Art

Easy and quantitative measurements of bone strength are desired for diagnosing bone metabolic diseases such as osteoporosis, judging fracture risk, and quantitatively diagnosing bone union after treatment of bone fracture.

The evaluation of bone formation and bone union depends largely on an X-ray photograph, but it is difficult to quantitatively diagnose the bone strength with an X-ray photograph. As a method of measuring bone strength, conventionally, there is known a strength test of a sample bone of a measurement target. However, in this method, it is necessary to apply an extraction operation of a sample bone, and thus, the method is invasive. As a method of measuring an amount of bone and a bone density, the use of devices such as X-ray CT and DXA (dual-energy x-ray absorptiometry) has been put in practice. However, these devices only measure the amount of bone and cannot provide an evaluation of the bone strength. Moreover, as X-rays are irradiated in these methods, these methods cannot be considered as non-invasive.

Other attempts to quantitatively evaluate the bone strength include a strain gauge method in which a strain gauge is mounted on an external fixtator and the strain of the external fixtator is measured, a vibration wave method in which a vibration is applied to a bone from the outside and a characteristic frequency is evaluated, and an acoustic emission method in which acoustic waves generated by a bone which has reached the yield stress are detected. These methods, however, have various problems in that there is a limitation to the treatment to which these methods can be applied, that invasion must be applied to the bone, and that the precision is insufficient.

Attempts have been made to analyze fine structures of the bone using ultrasound, but the relationship between the result of this analysis and the bone strength is not yet known (refer to, for example, Japanese Patent Laid-Open Publication No. Hei 9-84788).

US-2002/0103432 A1 discloses a non-invasive apparatus for assessing disorders of the musculoskeletal system. There, a plurality of ultrasonic beams is generated with respect to a bone in a subject and a plurality of echo signals corresponding to the ultrasonic beams is obtained. Further, edge detection algorithms are used to identify the surface of the bone and the portion of the bone surface which is in closest proximity to a transceiver unit. Also, surface points are tracked from a no-load state in which no load is applied to the bone to a loaded state in which a load is applied to the bone.

### SUMMARY OF THE INVENTION

There has been no device in the related art to non-invasively and quantitatively evaluate mechanical characteristics of bone such as bone strength. An advantage of the present invention is that an ultrasonic diagnostic apparatus for non-invasively and quantitatively evaluating mechanical characteristics of bone within a living body is provided.

According to one aspect of the present invention, there is provided an ultrasonic diagnostic apparatus comprising a transceiver unit which generates a plurality of ultrasonic beams with respect to a bone in a subject and obtains a plurality of echo signals corresponding to the ultrasonic beams; and a shape measurement unit which identifies a surface point corresponding to a bone surface for each of the echo signals and generates shape data of the bone surface based on the plurality of surface points obtained from the plurality of echo signals. According to this aspect of the present invention, the ultrasonic diagnostic apparatus further comprises a characteristic evaluation unit which evaluates a mechanical characteristic of the bone based on a change in the shape data when an external action is applied to the bone.

With such a structure, it is possible to non-invasively and quantitatively evaluate mechanical characteristics of the bone within a living body from shape data of a bone surface based on the echo signals.

According to another aspect of the present invention, it is preferable that, in the ultrasonic diagnostic apparatus, the transceiver unit generates the ultrasonic beams within a same cross section of the bone in the subject. According to another aspect of the present invention, it is preferable that, in the ultrasonic diagnostic apparatus, the shape measurement unit comprises a tracking unit which tracks the surface points from a state in which the external action is not applied to a state in which the external action is applied. According to another aspect of the present invention, it is preferable that, in the ultrasonic diagnostic apparatus, the shape measurement unit comprises a shape data generator unit which generates the shape data for each time phase from the state in which the external action is not applied and the state in which the external action is applied.

With such a structure, the transceiver unit generates ultrasonic beams within the same cross section with respect to a bone in the subject and the bone surface points are tracked. Because of this, a bone surface is always detected within the same cross section during the entire measurement period. Therefore, it is possible to obtain very precise shape data. According to another aspect of the present invention, it is preferable that, in the ultrasonic diagnostic apparatus, the shape data generator unit generates, as the shape data, an interpolation line connecting the plurality of surface points for each time phase. With such a structure, it is possible to evaluate a bone on an arbitrary point on the generated interpolation line.

According to another aspect of the present invention, it is preferable that, in the ultrasonic diagnostic apparatus, the characteristic evaluation unit overlaps the interpolation line corresponding to the state in which the external action is not applied and the interpolation line corresponding to the state in which the external action is applied by overlapping one of the interpolation lines on the other to correct a displacement due to a movement of the bone between the two interpolation lines and evaluates the mechanical characteristic of the bone based on the two corrected interpolation lines. With such a structure, even when the bone moves with respect to the transceiver unit during measurement, it is possible to evaluate while correcting the displacement due to the movement of the bone.

According to another aspect of the present invention, there is provided an ultrasonic diagnostic apparatus comprising a transceiver unit which generates a plurality of ultrasonic beams with respect to a bone in a subject and obtains a plurality of echo signals corresponding to the ultrasonic beams; and a tracking unit which identifies a surface point corresponding to a bone surface for each of the echo signals and tracks the surface points from a no-load state in which no load is applied to the bone to a loaded state in which a load is applied to the bone.

According to this aspect of the present invention, the ultrasonic diagnostic apparatus further comprises an interpolation line generator unit which generates an interpolation line connecting a plurality of surface points for each time phase in the no-load state and in the loaded state. According to this aspect of the present invention, the ultrasonic diagnostic apparatus further comprises a translational displacement corrector unit which translates one of the interpolation line corresponding to the no-load state and the interpolation line corresponding to the loaded state such that one of the interpolation lines is over the other interpolation line and corrects a translational displacement component between the two interpolation lines.

According to this aspect of the present invention, the ultrasonic diagnostic apparatus further comprises a strain calculator unit which calculates an amount of strain of the bone in the loaded state based on the two interpolation lines in which the translational displacement component is corrected. According to another aspect of the present invention, it is preferable that the ultrasonic diagnostic apparatus further comprises a characteristic curve generator unit which generates a characteristic curve indicating a relationship between the load value and the amount of strain of the bone in the loaded state. According to another aspect of the present invention, it is preferable that the ultrasonic diagnostic apparatus further comprises a display unit which displays a cross sectional image of the bone based on the plurality of echo signal, wherein the display unit displays the interpolation line at a suitable position on the cross sectional image of the bone.

### BRIEF DESCRIPTION OF THE DRAWINGS

A preferred embodiment of the present invention will be described in detail based on the following figures, wherein:
Fig. 1 is a block diagram showing an overall structure of an ultrasonic diagnostic apparatus according to a preferred embodiment of the present invention;
Fig. 2 is a diagram schematically showing each echo signal;
Fig. 3 is a diagram for explaining tracking of a bone surface section by the echo signals;
Fig. 4 is a diagram for explaining the operation to cancel a translational displacement component;
Fig. 5 is a diagram showing a method of diagnosing the tibia using an ultrasonic diagnostic apparatus according to a preferred embodiment of the present invention;
Fig. 6 is a diagram showing a method of fixing a probe;
Fig. 7 is a diagram showing another method of fixing a probe;
Fig. 8 is a diagram showing another method of fixing a probe;
Fig. 9 is a diagram showing an example of a characteristic curve of a change with respect to time of load and strain, generated by a characteristic curve generator unit;
Fig. 10 is a diagram showing an example of a characteristic curve showing a relationship between a load and strain, generated by a characteristic curve generator unit;
Fig. 11 is a diagram showing another example of a characteristic curve showing a change with respect to time of a load and strain, generated by a characteristic curve generator unit; and
Fig. 12 is a diagram showing a hysteresis characteristic curve of a load and strain, generated by a characteristic curve generator unit.

### DESCRIPTION OF PREFERRED EMBODIMENT

A preferred embodiment of the present invention will now be described referring to the drawings.

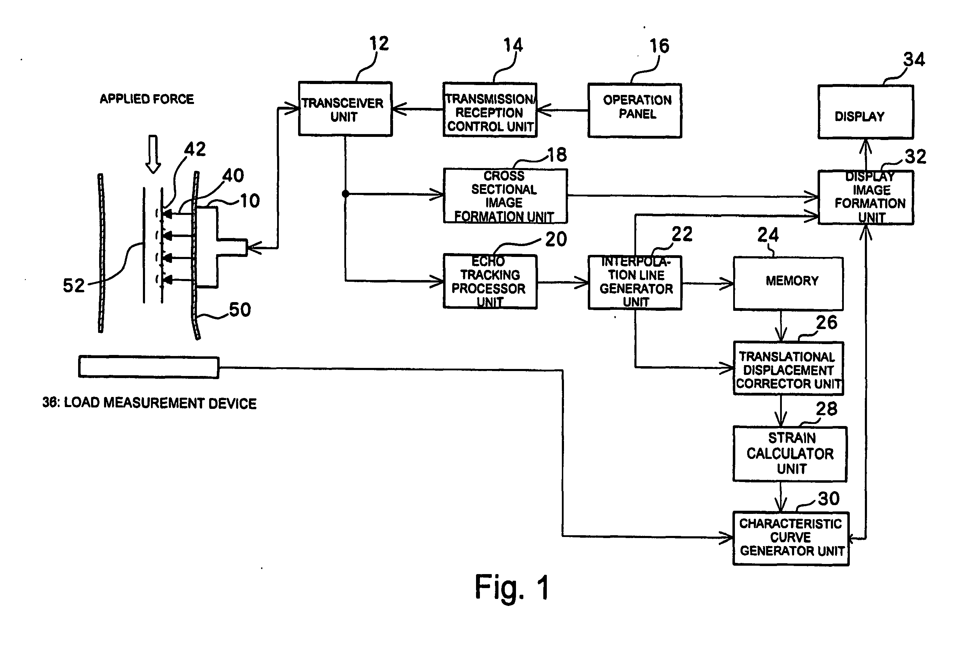

Fig. 1 shows a preferred embodiment of an ultrasonic diagnostic apparatus according to the present invention. Fig. 1 is a block diagram showing an overall structure of the ultrasonic diagnostic apparatus. A probe 10 is an ultrasonic probe used in contact with a surface of the body of a subject 50. Alternatively, an ultrasonic probe which is inserted into the subject may be used. The probe 10 transmits and receives ultrasonic beams 40 to and from a bone 52 within the body of the subject 50. Tracking points 42 which are set on the bone 52 will be described later. As the probe 10, it is desirable to use a linear electronic scan probe (linear array probe) which electronically scans the ultrasonic beam 40.

A transceiver unit 12 controls the probe 10 and electronically scans the ultrasonic beam 40 on a cross sectional surface (a cut surface of a subject in Fig. 1). When the probe 10 is a linear probe, for example, sequential electronic scanning of 120 ultrasonic beams 40 (Fig. 1 only shows 4 ultrasonic beams for echo tracking which will be described later) is applied and an echo signal is obtained for each ultrasonic beam 40. The plurality of obtained echo signals are output to a cross sectional image formation unit 18 and the cross sectional image formation unit 18 forms a cross sectional image (B mode image) of the bone based on the plurality of echo signals.

The echo signal obtained in the transceiver unit 12 is also output to an echo tracking processor unit 20. The echo tracking processor unit 20 applies an echo tracking process in which the bone surface section is extracted fromeach echo signal and is tracked. For the echo tracking process, for example, a technique detailed in Japanese Patent Laid-Open Publication No. 2001-309918 is used. For the echo tracking process, for example, 4 tracking echo signals are used. The tracking echo signals may be selected from among the echo signals used for forming a cross sectional image (for example, 120 echo signals) or may alternatively be obtained by interrupting formation of the cross sectional image and obtaining the 4 tracking echo signals.

The 4 ultrasonic beams 40 shown in Fig. 1 correspond to the 4 tracking echo signals. An examiner inputs an instruction related to transmission/reception of the ultrasound through an operation panel 16 to a transmission/reception control unit 14 and the transmission/reception control unit 14 controls the transceiver unit 12 based on the instruction from the examiner. With this structure, ultrasonic beams 40 for obtaining tracking echo signals are transmitted to a diagnosis site on the bone surface according to the instruction from the examiner. In the transmission/reception of ultrasound, a strong reflected wave is obtained from the bone surface. Therefore, the echo signals obtained from within the body of the diagnosis target (subject) are obtained as having large amplitude in a portion corresponding to the bone surface.

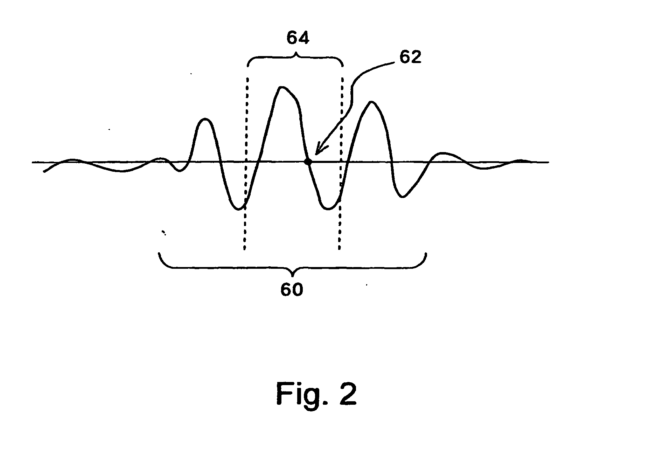

Fig. 2 is a diagram schematically showing a bone surface section of the echo signals. As shown in Fig. 2, each echo signal includes a range 60 in which the echo signal has a large amplitude corresponding to the bone surface. When the bone surface section is considered simply as a portion with a large amplitude, it is unclear as to which portion in the range 60 corresponds to the surface section and, as a result, there is an error of approximately the range 60. In the echo tracking process, a zero-cross point 62 is detected as a representative of each echo signal and the detected zero-cross point 62 is tracked to significantly increase the precision of the bone surface position.

The zero-cross point 62 is detected as a time, within a tracking gate period 64, at which the polarity of the echo signal is inverted from positive to negative or from negative to positive. In Fig. 2, the time at which the polarity of the echo signal is inverted from positive to negative is the zero-cross point 62. When the zero-cross point 62 is detected, a new tracking gate is set with the detected zero-cross point 62 as its center. In the echo signal obtained at the next time, a zero-cross point 62 is detected within the newly set tracking gate period 64. In this manner, a zero-cross point 62 is tracked as the bone surface point for each echo signal.

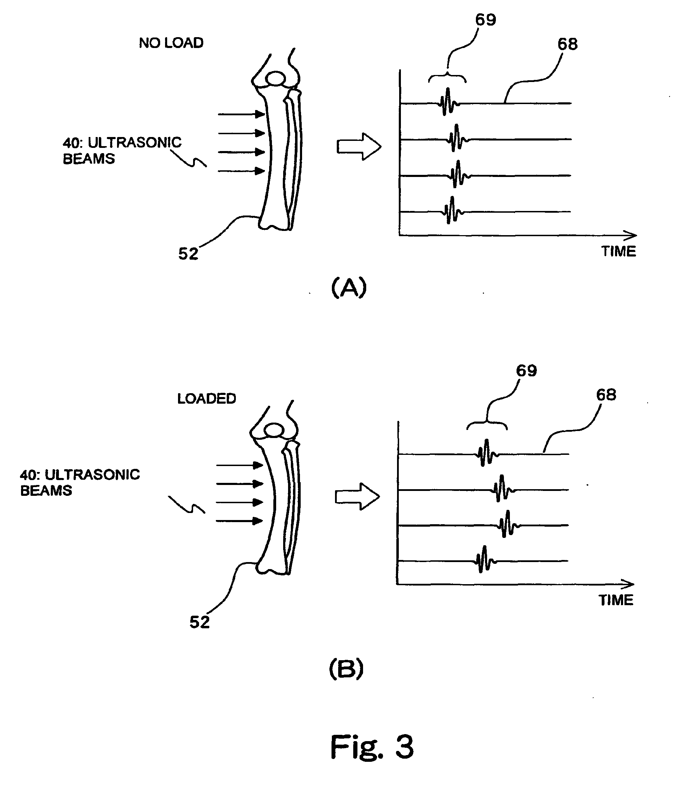

Fig. 3 is a diagram for explaining tracking of a bone surface section by 4 echo signals. In an evaluation of the mechanical characteristics of bone using the ultrasonic diagnostic apparatus of the present invention, a shape of the bone is compared between a state in which no load is applied to the bone (no-load state) and a state in which a load is applied (loaded state). Fig. 3 shows tracking in each of the no-load state and the loaded state.

Fig. 3 (A) shows tracking with respect to the bone 52 in the no-load state. Echo signals 68 corresponding to 4 ultrasonic beams 40 applied toward the bone 52 indicate a large amplitude (an amplitude maximum portion 69) at a section corresponding to the bone surface. It is possible to know the shape of the bone surface based on the position of the amplitude maximum portion 69 in each echo signal 68 (obtained time of the waveform). Because the zero-cross point (reference numeral 62 in Fig. 2) is detected as a surface point within the amplitude maximum portion 69, the position of the bone surface is very precisely identified.

Fig. 3 (B) shows tracking with respect to the bone 52 in the loaded state. Similar to Fig. 3 (A), the shape of the bone surface can be known based on the echo signals 68 corresponding to the 4 ultrasonic beams 40. Because a load is applied, strain (bending of bone) of the bone 52 in Fig. 3 (B) is larger than that in Fig. 3 (A). Although an example configuration with 4 tracking echo signals is shown in Fig. 3, it is also possible to measure with a plurality of tracking echo signals, the number being different from 4.

Referring again to Fig. 1, a surface point which is tracked for each echo signal, that is, for each ultrasonic beam 40 in the echo tracking processor unit 20 is the tracking point 42. An interpolation line generator unit 22 generates an interpolation line connecting these tracking points 42. That is, by interpolating with a curve among a plurality of tracking points 42 using a spline interpolation or a least square interpolation, an interpolation line is calculated. By increasing the number of echo signals for echo tracking process it is possible to more precisely approximate the surface shape of the bone by the interpolation line.

When a mechanically specific section exists in a bone, an interpolation line is calculated considering the specific section. For example, when there is a fracture in the bone, it is possible to form interpolation lines for the portions of the bone separated by the bone fracture and to combine these two interpolation lines to form an interpolation line for the bone as a whole. The interpolation line is calculated for each time phase and is output to a memory 24, a translational displacement corrector unit 26, and a display image formation unit 32. The translational displacement corrector unit 26 cancels a translational displacement component between an interpolation line corresponding to a time phase in the no-load state stored in the memory 24 and an interpolation line corresponding to a time phase in the loaded state output from the interpolation line generator unit 22.

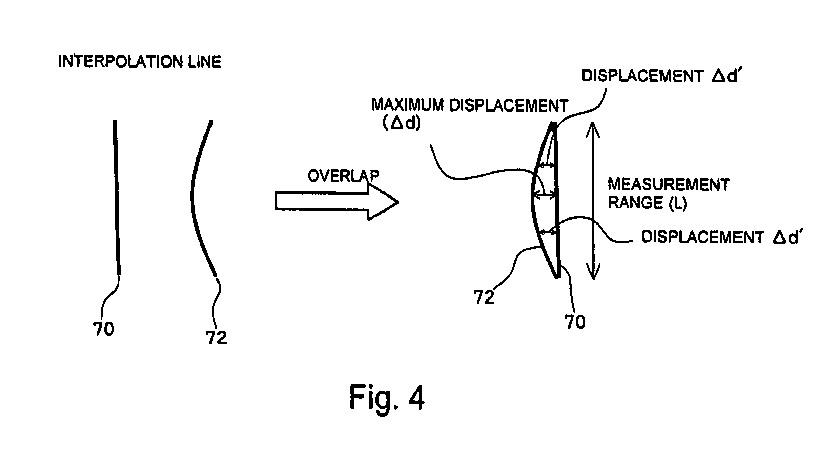

Fig. 4 is a diagram for explaining a cancel operation of the translational displacement component in the translational displacement corrector unit 26. The translational displacement corrector unit 26 overlaps the interpolation line corresponding to the no-load state (no-load-state interpolation line 70) and the interpolation line corresponding to the loaded state (loaded-state interpolation line 72) on each other to cancel the translational displacement component. The overlapping of the interpolation lines is performed, for example, by matching the ends of the no-load-state interpolation line 70 and the loaded-state interpolation line 72. By performing the overlapping operation, it is possible to cancel out the translational displacement between interpolation lines due to a movement of the bone during measurement. With this structure, an amount of strain of the bone is very precisely extracted from a difference between the no-load-state interpolation line 70 and the loaded-state interpolation line 72 which are overlapped. An amount ε of strain of the bone is defined, based on a maximum displacement Δd between the overlapped no-load-state interpolation line 70 and loaded-state interpolation line 72 and a measurement range (length of the no-load-state interpolation line 70) L, as ε = Δd/L. When a displacement at an arbitrary point between the no-load-state interpolation line 70 and the loaded-state interpolation line 72 is Δd', it is possible to calculate ε' = Δd'/L as the amount of strain of the bone.

Referring again to Fig. 1, a strain calculator unit 28 calculates an amount ε of strain of the bone and outputs to a characteristic curve generator unit 30. The characteristic curve generator unit 30 generates a characteristic curve regarding the strain of the bone based on a load value output by a load measurement device 36 and an amount of strain of bone output by the strain calculator unit 28.

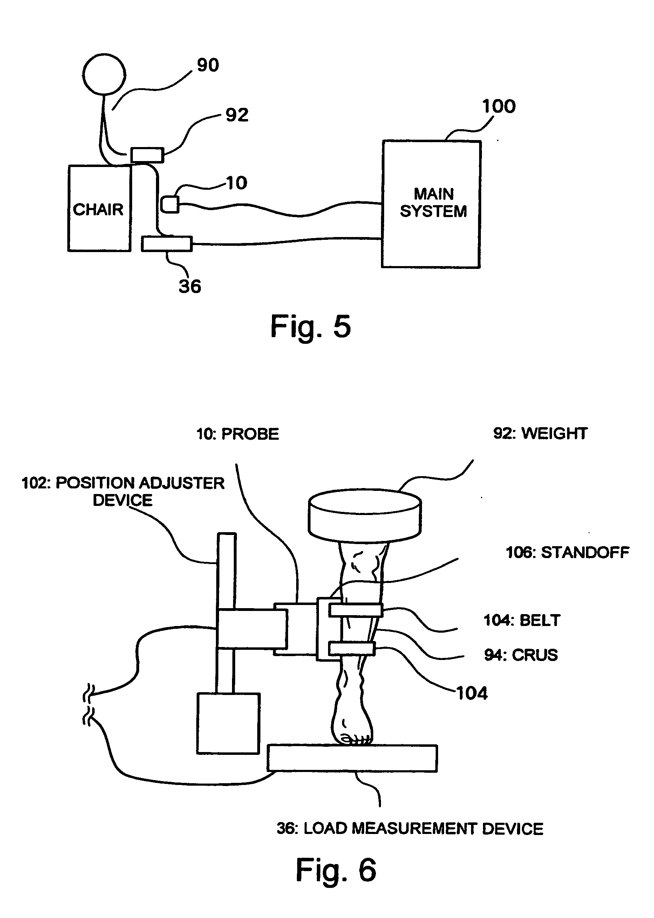

A method for diagnosing mechanical characteristics of a bone using the ultrasonic diagnostic apparatus according to the preferred embodiment of the present invention will now be described. Fig. 5 is a diagram showing a method for diagnosing the tibia using the ultrasonic diagnostic apparatus of Fig. 1. A weight 92 is placed on the lap of a subject 90 who is sitting on a chair so that a load due to the weight 92 is applied to the tibia. The load value due to the weight 92 is measured by the load measurement device 36 and is output to a main system 100 of the ultrasonic diagnosis device. The probe 10 is fixed by a probe fixer which is not shown and transmits and receives ultrasound to and from the crus (a site of the tibia).

A positional relationship among the probe 10, crus, and probe fixer is shown in Fig. 6. The probe 10 is supported by a position adjuster device 102 which functions as a probe fixer and placed in a predetermined position. A load due to the weight 92 is applied to the crus 94 of the subject and the load value is measured by the load measurement device 36. A standoff 106 is mounted on the crus 94 with a belt 104. The standoff 106 is a gel-like medium which allows ultrasound to transmit through and has an acoustic impedance which is close to that of a living body. The probe 10 is placed in contact with the standoff 106 and transmits and receives ultrasound to and from the crus 94 of the subject via the standoff 106, to evaluate the mechanical characteristics of the tibia or fibula.

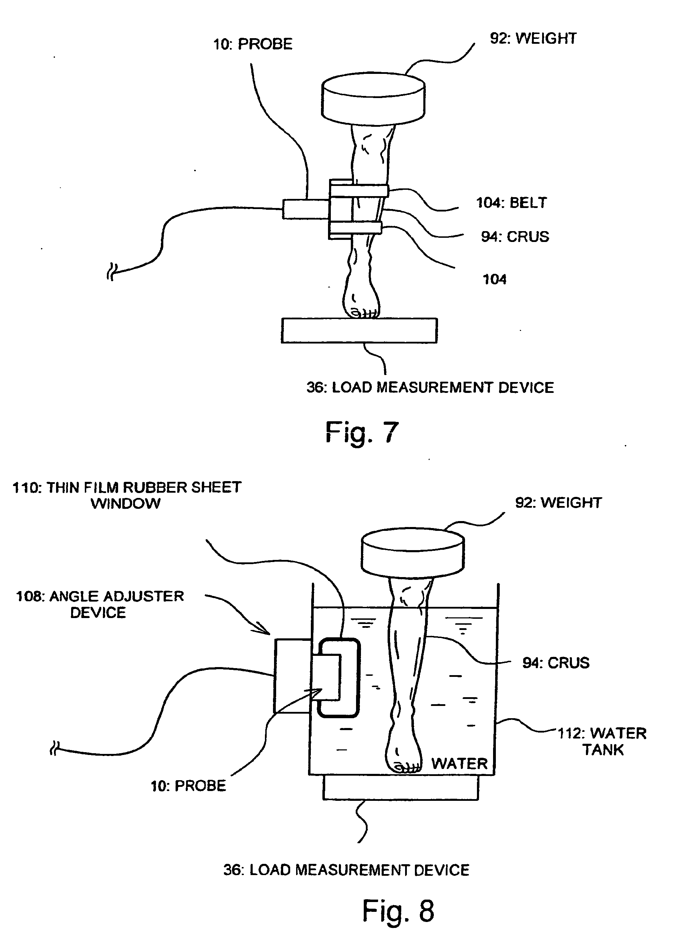

Figs. 7 and 8 show other examples of methods for fixing the probe 10. In the example configuration of Fig. 7, the probe 10 is placed in contact with the crus 94 using the belt 104. In the example configuration of Fig. 8, the subject places the crus 94 in a water tank 112 and the probe 10 is fixed by an angle adjuster device 108 mounted on the water tank 112. The probe 10 is covered by a thin film rubber sheet window 110 and transmits and receives ultrasound to and from the crus 94 via water. In a practical setting, it is desirable that a fixing device which is not shown is provided so that the position of the crus 94 with respect to the probe 10 does not change. In the example configurations of Figs. 7 and 8 also, the load value due to the weight 92 is measured by a load measurement device 36.

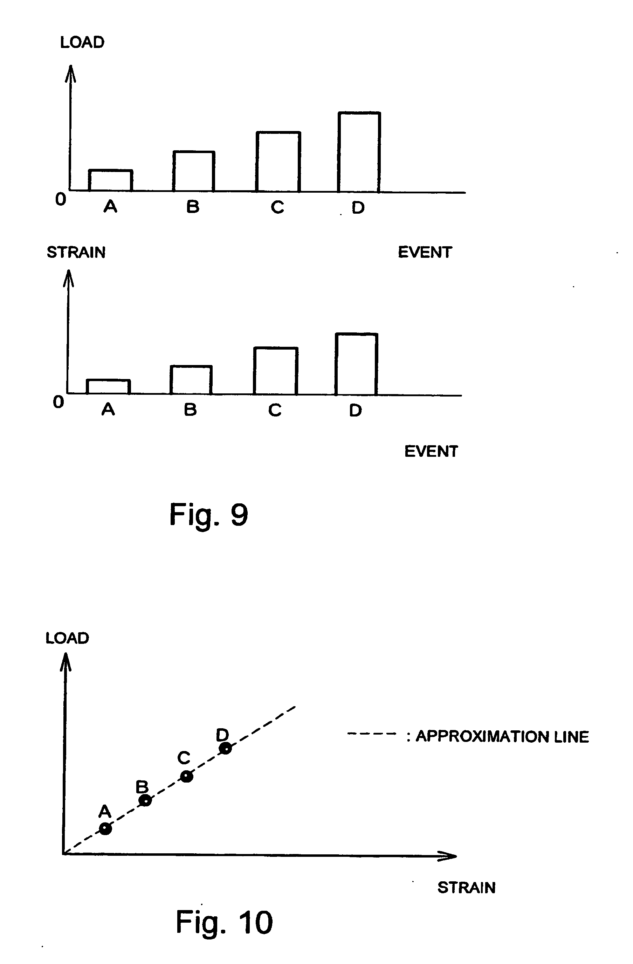

By changing the weight 92, it is possible to measure strain for each load value. Based on data obtained through this process, the characteristic curve generator unit 30 generates a characteristic curve. Figs. 9 and 10 show examples of characteristic curves generated by the characteristic curve generator unit 30. Fig. 9 is a diagram showing each of a load value and an amount of strain of bone with the horizontal axis taken as an event (four stages of A, B, C, and D). It can be seen that, when the load value is increased stepwise in the order of events A, B, C, and D, the amount of strain of bone correspondingly increases in a stepwise manner.

Fig. 10 is a diagram showing a relationship between the amount of strain of bone and the load value shown in Fig. 9, with the horizontal axis representing the amount of strain of bone and the vertical axis representing the load value. Fig. 10 also shows an approximation line calculated with data discretely obtained in the four stages of A, B, C, and D. The characteristic curves shown in Figs. 9 and 10 are displayed on a display (reference numeral 34 of Fig. 1) through a display image formation unit (reference numeral 32 of Fig. 1).

As a mechanical characteristic of bone, for example, it is possible to quantify (load)/(strain) which is a slope of the approximation line in Fig. 10, as a measure of the rigidity of the bone. For example, when an average value for healthy subjects is A and the value for the patient is B, it is determined that the rigidity of the bone of the diagnosis target (subject) is normal when the value of B is within a predetermined range estimated from the value of A and that the rigidity of the bone of the diagnosis target is abnormal when the value of B is outside the range. It is also possible to evaluate the rigidity of the bone from a difference, (B - A).

It is also possible to evaluate a strength of bone by applying a weight load which is an external action to the bone and quantifying plastic deformation of the bone after the weight is removed. As a concrete example, it is possible to compare the position of a surface of the bone before a load is applied and the position of the surface of the bone after a load is applied and then removed. It is possible to determine that the strength of the bone is normal when the difference is within a predetermined range and that the strength of the bone is abnormal when the difference is outside the range.

Other methods for applying a load to the tibia include standing on one foot, standing on both feet, and cycle load by walking. In any of these methods, the load value can be measured by the load measurement device (reference numeral 36 in Fig. 1) similar to the above-described configuration.

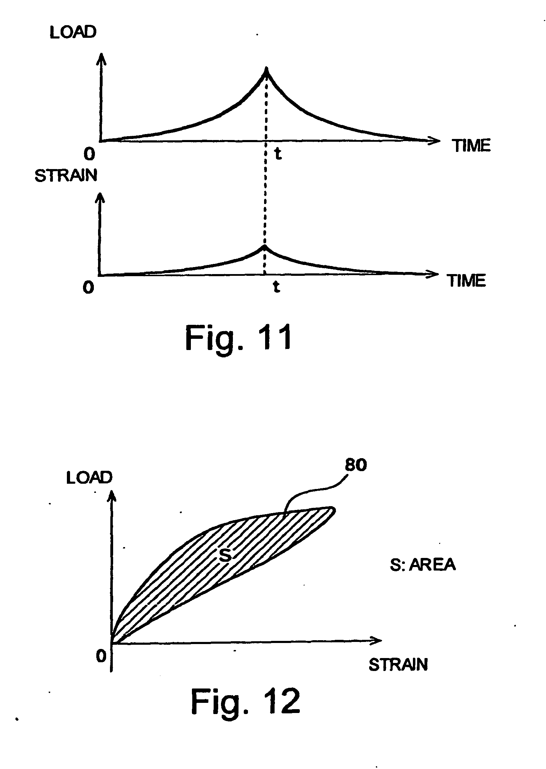

Figs. 11 and 12 show examples of characteristic curves generated by the characteristic curve generator unit 30 in the case of a cycle load. Fig. 11 is a characteristic curve showing each of the load value and amount of strain of the bone with the horizontal axis representing time. Fig. 11 shows that when the load value is gradually increased from time 0 to time t, the amount of strain correspondingly gradually increases from time 0 to time t. It can also be seen that when the load value is gradually reduced after time t, the amount of strain is also gradually reduced. The characteristic curve of Fig. 11 is displayed on a display (reference numeral 34 in Fig. 1) through a display image formation unit (reference numeral 32 in Fig. 1). The examiner can read the amount of strain of the bone corresponding to a load value from the characteristic curve displayed on the display 34.

Fig. 12 shows a characteristic curve in which the horizontal axis represents an amount of strain of the bone and the vertical axis represents a load value. There is a hysteresis characteristic between the amount of load to the bone and the amount of strain of the bone. In other words, an increase characteristic of the amount of strain of the bone when the load value is gradually increased to the maximum load value and a decrease characteristic of the amount of strain of the bone when the load is gradually reduced from the maximum load value would not be the same curve. Fig. 12 shows a characteristic when the load value is increased to a maximum load value and then reduced from the maximum load value. The area of a region 80 reflects the hysteresis characteristic between the load value and the amount of strain. The characteristic curve shown in Fig. 12 is displayed on the display (reference numeral 34 of Fig. 1) through the display image formation unit (reference numeral 32 of Fig. 1).

Referring again to Fig. 1, the display image formation unit 32 forms a display image based on the cross sectional image of the bone formed in the cross sectional image formation unit 18 and the characteristic curve generated by the characteristic curve generator unit 30 and displays the formed image on the display 34. The cross sectional image and the characteristic curve are, for example, switched and displayed based on an instruction by the examiner. Alternatively, the cross sectional image and the characteristic curve may besimultaneously displayed. Moreover, it is also possible to display the interpolation line calculated by the interpolation line generator unit 22, overlapped over the cross sectional image of the bone.

The mechanical characteristic thus obtained such as the amount of strain or strength of bone is an important measure of a quantitative evaluation of bone union and significantly contributes as objective and reliable base data of diagnosis in judgment of effects by an agent to an increase in the bone strength, removal of a fixator/implant, and instruction of degree of load to a patient.

The diagnosis target bone of the ultrasonic diagnostic apparatus according to the present invention is not limited to the tibia and the fibula, and may alternatively applied to, for example, femur and armbones. When the femur is to be targeted, it is possible to place, on a load measurement device, a diagnosis target wrapping around a weight and diagnose with the probe contacting the femur, to measure the load value applied to the femur and the shape of the femur. When the arm bones are targeted, it is possible to contact the probe on the arm of the subject and to diagnose while the arm is loaded by the subject pushing, with the arm, a load measurement device mounted on a wall. Alternatively, it is also possible for the subject to place his arm on the load measurement device placed on the floor and to diagnose the loaded state in a position of "push-up" . In this manner, the ultrasonic diagnostic apparatus of the present invention can be applied to the bones of various regions in the subject.

A diagnosis using the ultrasonic diagnostic apparatus of the present invention is advantageous in that the diagnosis can be applied independent of the method of treatment of bone fracture.

As described, with the ultrasonic diagnostic apparatus of the present invention, it is possible to non-invasively and quantitatively evaluate a mechanical characteristic of the bone within a living body.

## Claims

1. An ultrasonic diagnostic apparatus comprising:
a transceiver unit (12) which generates a plurality of ultrasonic beams with respect to a bone (52) in a subject (50) and obtains a plurality of echo signals corresponding to the ultrasonic beams; and
a shape measurement unit which identifies a surface point corresponding to a bone surface for each of the echo signals and generates shape data of the bone surface based on the plurality of surface points obtained from the plurality of echo signals,
**characterized by**
a characteristic evaluation unit which evaluates a mechanical characteristic of the bone based on a change in the shape data when an external action is applied to the bone.

2. An ultrasonic diagnostic apparatus according to Claim 1, wherein
the transceiver unit (12) generates the ultrasonic beams within a same cross section of the bone (52) in the subject (50).

3. An ultrasonic diagnostic apparatus according to Claim 2, wherein
the shape measurement unit comprises a tracking unit which tracks the surface points from a state in which the external action is not applied to a state in which the external action is applied.

4. An ultrasonic diagnostic apparatus according to Claim 3, wherein
the shape measurement unit comprises a shape data generator unit which generates the shape data for each time phase in the state in which the external action is not applied and the state in which the external action is applied.

5. An ultrasonic diagnostic apparatus according to Claim 4, wherein
the shape data generator unit generates, as the shape data, an interpolation line connecting the plurality of surface points for each time phase.

6. An ultrasonic diagnostic apparatus according to Claim 5, wherein
the characteristic evaluation unit overlaps the interpolation line corresponding to the state in which the external action is not applied and the interpolation line corresponding to the state in which the external action is applied by overlapping one of the interpolation lines on the other to correct a displacement due to a movement of the bone between the two interpolation lines and evaluates the mechanical characteristic of the bone based on the two corrected interpolation lines.

7. An ultrasonic diagnostic apparatus comprising:
a transceiver unit (12) which generates a plurality of ultrasonic beams with respect to a bone (52) in a subject (50) and obtains a plurality of echo signals corresponding to the ultrasonic beams; and
a tracking unit which identifies a surface point corresponding to a bone surface for each of the echo signals and tracks the surface points from a no-load state in which no load is applied to the bone to a loaded state in which a load is applied to the bone,
**characterized by**
an interpolation line generator unit (22) which generates an interpolation line connecting a plurality of the surface points for each time phase in the no-load state and in the loaded state,
a translational displacement corrector unit (26) which translates one of the interpolation line corresponding to the no-load state and the interpolation line corresponding to the loaded state such that one of the interpolation line is over the other interpolation line and corrects a translational displacement component between the two interpolation lines, and a
a strain calculator unit (28) which calculates an amount of strain of the bone in the loaded state based on the two interpolation lines in which the translational displacement component is corrected.

8. An ultrasonic diagnostic apparatus according to Claim 7, wherein
the tracking unit detects a zero-cross point as the surface point and tracks the detected zero-cross point.

9. An ultrasonic diagnostic apparatus according to Claim 8, wherein
the zero-cross point is detected as a time at which a polarity of the echo signal is inverted from positive to negative or from negative to positive within a tracking gate period.

10. An ultrasonic diagnostic apparatus according to Claim 7, wherein
the interpolation line generator unit (22) generates an interpolation line using a spline interpolation.

11. An ultrasonic diagnostic apparatus according to Claim 7, wherein
the interpolation line generator unit (22) generates an interpolation line using a least square interpolation.

12. An ultrasonic diagnostic apparatus according to Claim 7, further comprising:
a characteristic curve generator unit (30) which generates a characteristic curve indicating a relationship between the load value and the amount of strain of the bone in the loaded state.

13. An ultrasonic diagnostic apparatus according to Claim 12, wherein
the characteristic curve generator unit (30) generates a characteristic curve in which one axis represents time and another axis represents the load value.

14. An ultrasonic diagnostic apparatus according to Claim 12, wherein
the characteristic curve generator unit (30) generates a characteristic curve in which one axis represents time and another axis represents the amount of strain of the bone.

15. An ultrasonic diagnostic apparatus according to Claim 12, wherein
the characteristic curve generator unit (30) generates a characteristic curve in which one axis represents the amount of strain of the bone and another axis represents the load value.

16. An ultrasonic diagnostic apparatus according to Claim 12, further comprising:
a display unit (34) which displays a cross sectional image of the bone based on the plurality of echo signals, wherein the display unit (34) displays the interpolation line at a suitable position on the cross sectional image of the bone (52).

## Patentansprüche

1. Ultraschalldiagnosegerät mit
einer Sender-Empfänger-Einheit (12), die eine Vielzahl von Ultraschallstrahlen bezüglich eines Knochens (52) in einem Subjekt (50) generiert und eine Vielzahl von den Ultraschallstrahlen entsprechenden Echosignalen empfängt, und
einer Formmesseinheit, die einen einer Knochenoberfläche entsprechenden Oberflächenpunkt für jedes der Echosignale identifiziert und Formdaten der Knochenoberfläche auf der Basis der Vielzahl von durch die Vielzahl der Echosignale erhaltenen Oberflächenpunkte generiert,
**gekennzeichnet durch**
eine charakteristische Auswerteeinheit, die eine mechanische Charakteristik des Knochens auf der Basis einer Veränderung in den Formdaten, wenn eine externe Einwirkung auf den Knochen ausgeübt wird, auswertet.

2. Ultraschalldiagnosegerät nach Anspruch 1,
wobei
die Sender-Empfänger-Einheit (12) die Ultraschallstrahlen innerhalb einer selben Querschnittsfläche des Knochens (52) in dem Subjekt (50) generiert.

3. Ultraschalldiagnosegerät nach Anspruch 2,
wobei
die Formmesseinheit eine Abtasteinheit umfasst, die die Oberflächenpunkte von einem Zustand, in dem die externe Einwirkung nicht ausgeübt wird, bis zu einem Zustand, in dem die externe Einwirkung ausgeübt wird, abtastet.

4. Ultraschalldiagnosegerät nach Anspruch 3,
wobei
die Formmesseinheit eine Formdatengeneriereinheit umfasst, die die Formdaten für jede Zeitphase in dem Zustand, in dem die externe Einwirkung nicht ausgeübt wird und in dem Zustand, in dem die externe Einwirkung ausgeübt wird, generiert.

5. Ultraschalldiagnosegerät nach Anspruch 4,
wobei
die Formdatengeneriereinheit eine Interpolationslinie, als die Formdaten, generiert, die die Vielzahl von Oberflächenpunkten für jede Zeitphase verbindet.

6. Ultraschalldiägnosegerät nach Anspruch 5,
wobei
die charakteristische Auswerteeinheit die Interpolationslinie entsprechend dem Zustand, in dem die externe Einwirkung nicht ausgeübt wird und die Interpolationslinie entsprechend dem Zustand, in dem die externe Einwirkung ausgeübt wird, übereinander legt, durch Übereinanderlegen einer der Interpolationslinien auf die andere, um eine Verschiebung aufgrund einer Bewegung des Knochens zwischen den zwei Interpolationslinien zu korrigieren und die mechanische Charakteristik des Knochens auf Grundlage der beiden korrigierten Interpolationslinien auswertet.

7. Ultraschalldiagnosegerät mit
einer Sender-Empfänger-Einheit (12), die eine Vielzahl von Ultraschallstrahlen bezüglich eines Knochens (52) in einem Subjekt (50) generiert und eine Vielzahl von den Ultraschallstrahlen entsprechenden Echosignalen empfängt, und
einer Abtasteinheit, die einen einer Knochenoberfläche entsprechenden Oberflächenpunkt für jedes der Echosignale identifiziert und die Oberflächenpunkte von einem Nichtbelastungszustand, in dem keine Belastung auf den Knochen ausgeübt wird, bis zu einem belasteten Zustand, in dem eine Belastung auf den Knochen ausgeübt wird, abtastet,
**gekennzeichnet durch**
eine Interpolationsliniengeneriereinheit (22), die eine Interpolationslinie, die eine Vielzahl von Oberflächenpunkten für jede Zeitphase in dem Nichtbelastungszustand und in dem belasteten Zustand verbindet, generiert,
einer Längsverschiebungskorrektureinheit (26), die die Interpolationslinie entsprechend dem Nichtbelastungszustand oder die Interpolationslinie entsprechend dem belasteten Zustand in der Weise umsetzt, dass eine der Interpolationslinien über der anderen ist, und eine Längsverschiebungskomponente zwischen den beiden Interpolationslinien korrigiert, und
eine Deformationsberechnungseinheit (28), die eine Größe der Deformation des Knochens in dem belasteten Zustand auf der Basis der zwei Interpolationslinien, bei denen die Längsverschiebungskomponente korrigiert ist, berechnet.

8. Ultraschalldiagnosegerät nach Anspruch 7,
wobei
die Abtasteinheit einen Nulldurchgangspunkt als den Oberflächenpunkt erkennt und den erkannten Nulldurchgangspunkt abtastet.

9. Ultraschalldiagnosegerät nach Anspruch 8,
wobei
der Nulldurchgangspunkt als eine Zeit erkannt wird, bei der die Polarität des Echosignals innerhalb einer Abtasttorperiode von Positiv zu Negativ oder von Negativ zu Positiv invertiert ist.

10. Ultraschalldiagnosegerät nach Anspruch 7,
wobei
die Interpolationsliniengeneriereinheit (22) eine Interpolationslinie unter Verwendung einer Spline-Interpolation generiert.

11. Ultraschalldiagnosegerät nach Anspruch 7,
wobei
die Interpolationsliniengeneriereinheit (22) eine Interpolationslinie unter Verwendung mindestens einer quadratischen Interpolation generiert.

12. Ultraschalldiagnosegerät nach Anspruch 7,
weiterhin mit
einer charakteristischen Kurve-Generiereinheit (30), die eine charakteristische Kurve generiert, die eine Beziehung zwischen dem Belastungswert und der Größe der Deformation des Knochens in dem belasteten Zustand anzeigt.

13. Ultraschalldiagnosegerät nach Anspruch 12,
wobei
die charakteristische Kurve-Generiereinheit (30) eine charakteristische Kurve generiert, bei der eine Achse die Zeit darstellt und eine andere Achse den Belastungswert darstellt.

14. Ultraschalldiagnosegerät nach Anspruch 12,
wobei
die charakteristische Kurve-Generiereinheit (30) eine charakteristische Kurve generiert, bei der eine Achse die Zeit darstellt und eine andere Achse die Größe der Deformation des Knochens darstellt.

15. Ultraschalldiagnosegerät nach Anspruch 12,
wobei
die charakteristische Kurve-Generiereinheit (30) eine charakteristische Kurve generiert, bei der eine Achse die Größe der Deformation des Knochens darstellt und eine andere Achse den Belastungswert darstellt.

16. Ultraschalldiagnosegerät nach Anspruch 12,
weiterhin mit
einer Anzeigeeinheit (34), die ein Querschnittsbild des Knochens auf der Basis der Vielzahl von Echosignalen darstellt, wobei die Anzeigeeinheit (34) die Interpolationslinie an einer geeigneten Position auf dem Querschnittsbild des Knochens (52) darstellt.

## Revendications

1. Appareil de diagnostic à ultrasons comprenant :
une unité d'émission-réception (12) qui génère une pluralité de faisceaux d'ultrasons concernant un os (52) chez un sujet (50) et reçoit une pluralité de signaux d'écho correspondant aux faisceaux d'ultrasons ; et
une unité de mesure de forme qui identifie un point de surface correspondant à une surface de l'os pour chacun des signaux d'écho et qui génère des données de forme de la surface de l'os à partir de la pluralité de points de surface obtenus de la pluralité de signaux d'écho,
***caractérisé par***
une unité d'évaluation de caractéristique qui évalue une caractéristique mécanique de l'os à partir d'une modification des données de forme lorsqu'une action extérieure est appliquée sur l'os.

2. Appareil de diagnostic à ultrasons selon la revendication 1, dans lequel
l'unité d'émission-réception (12) génère les faisceaux d'ultrasons dans une même section transversale de l'os (52) chez le sujet (50).

3. Appareil de diagnostic à ultrasons selon la revendication 2, dans lequel
l'unité de mesure de forme comprend une unité de suivi qui suit les points de surface depui un état dans lequel l'action extérieure n'est pas appliquée, jusqu'à un état dans lequel l'action extérieure est appliquée.

4. Appareil de diagnostic à ultrasons selon la revendication 3, dans lequel
l'unité de mesure de forme comprend une unité génératrice de données de forme qui génère les données de forme pour chaque phase temporelle dans l'état dans lequel l'action extérieure n'est pas appliquée et dans l'état dans lequel l'action extérieure est appliquée.

5. Appareil de diagnostic à ultrasons selon la revendication 4, dans lequel
l'unité génératrice de données de forme génère, comme données de forme, une ligne d'interpolation reliant la pluralité de points de surface pour chaque phase temporelle.

6. Appareil de diagnostic à ultrasons selon la revendication 5, dans lequel
l'unité d'évaluation de caractéristiques superpose la ligne d'interpolation correspondant à l'état dans lequel l'action extérieure n'est pas appliquée et la ligne d'interpolation correspondant à l'état dans lequel l'action extérieure est appliquée en superposant l'une des lignes d'interpolation sur l'autre afin de corriger un déplacement dû à un mouvement de l'os entre les deux lignes d'interpolation et évalue la caractéristique mécanique de l'os à partir des deux lignes d'interpolation corrigées.

7. Appareil de diagnostic à ultrasons comprenant :
une unité d'émission-réception (12) qui génère une pluralité de faisceaux d'ultrasons concernant un os (52) chez un sujet (50) et reçoit une pluralité de signaux d'écho correspondant aux faisceaux d'ultrasons ; et
une unité de suivi qui identifie un point de surface correspondant à une surface de l'os pour chacun des signaux d'écho et qui suit les points de surface à partir d'un état hors charge dans lequel aucune charge n'est appliquée sur l'os, et jusqu'à un état sous charge dans lequel une charge est appliquée sur l'os,
**caractérisé par**
une unité (22) génératrice de lignes d'interpolation qui génère une ligne d'interpolation reliant une pluralité des points de surface pour chaque phase temporelle dans l'état hors charge et dans l'état sous charge,
une unité (26) correctrice de déplacement de translation qui translate l'une de la ligne d'interpolation correspondant à l'état hors charge et de la ligne d'interpolation correspondant à l'état sous charge de telle manière que l'une des lignes d'interpolation se trouve au-dessus de l'autre ligne d'interpolation, et corrige une composante de déplacement par translation entre les deux lignes d'interpolation, et
une unité (28) de calcul de déformation qui calcule une quantité de déformation de l'os dans l'état sous charge à partir des deux lignes d'interpolation, dans laquelle la composante de déplacement par translation est corrigée.

8. Appareil de diagnostic à ultrasons selon la revendication 7, dans lequel
l'unité de suivi détecte un point de passage par zéro comme point de surface et suit le point de passage par zéro détecté.

9. Appareil de diagnostic à ultrasons selon la revendication 8, dans lequel
le point de passage par zéro est détecté à un instant auquel une polarité du signal d'écho est inversée de positif à négatif ou de négatif à positif dans une période de porte de suivi.

10. Appareil de diagnostic à ultrasons selon la revendication 7, dans lequel
l'unité (22) génératrice de lignes d'interpolation génère une ligne d'interpolation à l'aide d'une interpolation par splines.

11. Appareil de diagnostic à ultrasons selon la revendication 7, dans lequel
l'unité (22) génératrice de lignes d'interpolation génère une ligne d'interpolation à l'aide d'une interpolation par la méthode des moindres carrés.

12. Appareil de diagnostic à ultrasons selon la revendication 7, comprenant de plus :
une unité (30) génératrice de courbe caractéristique qui génère une courbe caractéristique indiquant une relation entre la valeur de la charge et le taux de déformation de l'os dans l'état sous charge.

13. Appareil de diagnostic à ultrasons selon la revendication 12, dans lequel l'unité (30) génératrice de courbe caractéristique génère une courbe caractéristique dans laquelle un axe représente le temps et un autre axe représente la valeur de la charge.

14. Appareil de diagnostic à ultrasons selon la revendication 12, dans lequel l'unité (30) génératrice de courbe caractéristique génère une courbe caractéristique dans laquelle un axe représente le temps et un autre axe représente le taux de déformation de l'os.

15. Appareil de diagnostic à ultrasons selon la revendication 12, dans lequel l'unité (30) génératrice de courbe caractéristique génère une courbe caractéristique dans laquelle un axe représente le taux de déformation de l'os et un autre axe représente la valeur de la charge.

16. Appareil de diagnostic à ultrasons selon la revendication 12, comprenant de plus :
une unité d'affichage (34) qui affiche une image de la section transversale de l'os à partir de la pluralité de signaux d'écho, dans lequel l'unité d'affichage (34) affiche la ligne d'interpolation dans une position appropriée sur l'image de section transversale de l'os (52).
